(19)

Europäisches
Patentamt
European
Patent Office
Office européen
des brevets

(11)  **EP 2 627 229 B1**

(12)  **EUROPEAN PATENT SPECIFICATION**

(45) Date of publication and mention
of the grant of the patent:
**06.01.2016  Bulletin 2016/01**

(21) Application number: **10858470.7**

(22) Date of filing: **13.10.2010**

(51) Int Cl.:
*D21H 13/02* *(2006.01)*    *A47K 10/16* *(2006.01)*
*B32B 27/02* *(2006.01)*    *B32B 29/02* *(2006.01)*
*D04H 1/40* *(2012.01)*    *B32B 7/06* *(2006.01)*
*D04H 1/492* *(2012.01)*    *D21H 27/00* *(2006.01)*
*A61F 13/15* *(2006.01)*

(86) International application number:
**PCT/SE2010/051108**

(87) International publication number:
**WO 2012/050494 (19.04.2012 Gazette 2012/16)**

(54) **FLUSHABLE MOIST WIPE OR HYGIENE TISSUE**

FEUCHTES TOILETTENPAPIER ODER HYGIENETUCH, DAS IN DER TOILETTE WEGGESPÜLT
WERDEN KANN

LINGETTE OU PRODUIT D'HYGIÈNE EN PAPIER TISSU HUMIDE POUVANT ÊTRE JETÉS DANS
LES TOILETTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.08.2013  Bulletin 2013/34**

(73) Proprietor: **Sca Hygiene Products AB**
**405 03 Göteborg (SE)**

(72) Inventors:
• **STRANDQVIST, Mikael**
**S-437 36 Lindome (SE)**
• **GUNGNER, Susanne**
**S-446 36 Älvängen (SE)**

(74) Representative: **Valea AB**
**Box 1098**
**405 23 Göteborg (SE)**

(56) References cited:
**EP-A1- 0 303 528      EP-A1- 0 602 881
EP-A1- 1 138 474      EP-A1- 1 302 592
EP-A2- 0 421 163      EP-A2- 0 904 933
WO-A1-00/05065       WO-A1-02/22352
WO-A1-2010/019726    JP-A- H09 228 214
US-A- 6 028 018       US-A1- 2002 078 538
US-A1- 2003 073 367   US-A1- 2005 148 261
US-A1- 2009 286 437**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

FIELD OF THE INVENTION

The present invention refers to a moist wipe or hygiene tissue comprising a hydraulically entangled nonwoven material impregnated with a wetting composition. It is especially related to moist toilet paper and other wipes or hygiene tissue intended to be flushable in a sewer.

TECHNICAL BACKGROUND

Pre-moistened wipes or hygiene tissue, are commonly used for cleansing different parts of the human body. Examples of specific uses are for baby care, hand wiping, feminine care and as toilet paper or as a complement to toilet paper.

Since a long period of time often elapses from the time of manufacture of pre-moistened wipes until the time of use, they must have sufficient structural integrity for their intended wiping function during such period. Adding a wet strength agent to the wipe will provide such wet integrity. However, especially when used as toilet paper, there is a strong desire that the wipe or tissue can be flushed in the sewer without causing problems with blocked pipes and filters. Wipes or tissues having a high wet strength will not disintegrate or break up into small fibre clumps when flushed in conventional household toilet systems, which may cause plugging of the drainage system.

Most flushable pre-moistened toilet papers which are on the market today are flushable due to their small size. They can move along drainage and sewage pipes, but are not readily dispersible and may therefore cause problems with blocked pipes and filters.

It is also previously known to make flushable multiply pre-moistened wipes that will disperse when flushed in water. The individual plies in a multiply wipe can be made thinner and have lower strength properties than in a single ply wipe and therefore be more readily dispersible.

EP 0 903 997 discloses a water-dispersible wet wipe comprising at least two through-air-dried tissue sheets containing a wet strength agent and which are mechanically attached together by edge embossing. The edge embossing is sufficient to maintain the integrity of the wipe during use, but the wipe will readily disperse when flushed.

US 3,881,210 discloses a flushable wet wipe comprising two layers of fibrous material and a reinforcing layer of a water-dispersible thermoplastic film there between adhered to the fibrous material layers by embossing at spaced apart regions.

US 6,187,141 discloses a water-disintegratable fibrous sheet having a layered structure useful as a wet wipe. The layers may be held together by a water-soluble adhesive.

US 6,782,589 discloses a nonwoven laminate that is flushable. The laminate is formed by pattern hydroentangling together two nonwoven layers in alternating strongly bonded areas and unbonded areas. This will allow the laminate to loose its integrity and to delaminate in a sewage system.

SUMMARY OF THE INVENTION

The object of the present invention is to provide a moist wipe or hygiene tissue having good strength properties for the intended use but is readily dispersible when flushed in a sewage system. This object has according to the invention been solved by the fact that the moist wipe or hygiene tissue comprises a hydraulically entangled nonwoven material impregnated with a wetting composition, said nonwoven material containing 70-95%, by fibre weight, pulp fibres and 5-30%, by fibre weight, manmade fibres and/or natural fibres with a length of at least 6 mm, wherein said moist wipe or hygiene tissue comprises at least two plies of said hydraulically entangled nonwoven material, each ply having a basis weight between 30 and 50 g/m$^2$ and said plies are held together by frictional forces without additional mechanical bonding like embossing, needling and hydroentangling and without adhesive.

The manmade fibres or natural fibres may have a fibre length of up to 20 mm.

The moist wipe or hygiene tissue may contain not more than 0.1% by weight, as calculated on the dry weight, of a wet strength agent.

The dynamic friction coefficient ($\mu_D$) in wet condition between the plies of said hydraulically entangled nonwoven material may be at least 1.6, preferably at least 1.7. The moist wipe or hygiene tissue may contain at least one ply of tissue paper arranged between plies of said hydraulically entangled nonwoven material.

The moist wipe or hygiene tissue may be a moist toilet paper.

Each individual moist wipe or hygiene tissue may be folded in a way so that it comprises a leading edge panel, a trailing edge panel and optional intermediate panel(-s) there between, wherein the leading edge panel may be provided with an additional fold so as to provide a double-folded leading edge panel lip.

DESCRIPTION OF DRAWINGS

Embodiments of the invention will below be described with reference to the accompanying drawings.

Fig. 1 is a cross-sectional schematic illustration of an embodiment of a folded wipe.
Fig. 2 is a schematic view of an apparatus for measuring friction of a nonwoven material.

DETAILED DESCRIPTION

A premoistened wipe or hygiene tissue according to the invention comprises a hydroentangled nonwoven material impregnated with a wetting composition. The wetting composition may contain a major proportion of water and other ingredients depending on the intended use. Wetting compositions useful in moist wipes and hygiene tissue are well-known in the art.

Hydroentangling or spunlacing is a technique for forming a nonwoven web introduced during the 1970'ies, see e g CA patent no. 841 938. The method involves forming a fibre web, which is either drylaid or wetlaid, after which the fibres are entangled by means of very fine water jets under high pressure. Several rows of water jets are directed against the fibre web, which is supported by a movable foraminous support or a perforated drum. In this process the fibres entangle with one another providing sufficient bonding strength to the fibrous web without the use of chemical bonding agents. The entangled fibrous web is then dried. The fibres that are used in the material can be natural fibres, especially cellulosic pulp fibres, manmade staple fibres, and mixtures of pulp fibres and staple fibres. Spunlace materials can be produced with high quality to a reasonable cost and they possess a high absorption capacity.

The fibres used in the moist wipe or hygiene tissue according to the invention are at least 70-95%, by fibre weight, pulp fibres and 5-30% manmade fibres and/or natural fibres with a length of at least 6 mm. The manmade fibres may be synthetic, e g polyester, polyamide, polyethylene, polypropylene, polylactides and copolymers thereof or staple fibres of regenerated cellulose, such as viscose, rayon, lyocell or the like. The natural fibres with a fibre length of at least 6 mm may be cotton fibres, sisal, hemp, ramie, flax etc.

Cellulose pulp fibres can be selected from any type of pulp and blends thereof. Preferably the pulp is characterized by being entirely natural cellulosic fibres and can include wood fibres as well as cotton. Preferred pulp fibres are softwood papermaking pulp, although hardwood pulp and non-wood pulp, such as hemp and sisal may be used. The length of pulp fibres may vary from less than 1 mm for hardwood pulp and recycled pulp, to up to 6 mm for certain types of softwood pulp. Pulp fibres are advantageous to use since they are inexpensive, readily available and absorbent.

Short pulp fibres however have a rather poor capability to intertwine and entangle with each other during hydroentangling and are therefore usually mixed with longer fibres in order to produce a hydroentangled web with sufficient strength. These longer fibres having an average fibre length of at least 6 mm may be manmade fibres and/or natural fibres as mentioned above. Preferably said longer fibres have a fibre length of not more than 15 mm. The fineness of the longer fibres can vary between 0,3 dtex and 6 dtex.

The fibres are mixed and formed into a fibrous web. The fibrous web is either dry formed or wetlaid. In a wetlaid process the fibres are dispersed in a liquid, normally water, in a similar way as in a papermaking process and the dilute fibre dispersion is deposited on the foraminous support member where it is dewatered to form a continuous web-like material. The fibre dispersion may be diluted to any consistency that is typically used in conventional papermaking process. A foam forming process is a variant of a wet-laying process and a surfactant is added to the fibre dispersion, which is foamed, and the foamed fibre dispersion is deposited on the foraminous support. A very even fibre distribution is achieved in a foam forming process and it is also possible to use longer fibres than in a conventional wet-laying process.

The formed fibrous web is then subjected to hydroentanglement from several rows of nozzles, from which water jets at a high pressure are directed towards a fibrous web, while this is supported by the foraminous support member. The fibrous web is drained over suction boxes. Thereby, the water jets accomplish an entanglement of the fibrous web, i.e. an intertwining of the fibres. Appropriate pressures in the entanglement nozzles are adapted to the fibrous material, grammage of the fibrous web, etc. The water from the entanglement nozzles is removed via the suction boxes and is pumped to a water purification plant, and is then re-circulated to the entangling stations.

For a further description of the hydroentanglement or, as it is also called, spunlacing technology, reference is made e.g. to CA patent No. 841 938.

Hydroentangling may occur in one or several steps and from one side of the web or from both sides thereof. The web may be transferred to another foraminous support between two subsequent hydroentangling steps.

The entangled material is dewatered and brought to a drying station for drying before the finished material is reeled up and converted. Drying can be performed by blowing hot air through the fibrous web, by IR dryers or other non-compacting drying technique.

The web may be creped, embossed or otherwise textured to enhance softness of the product. Normally, working the web to enhance softness tends to reduce the wet strength of the web.

The entangled web is converted into wipes or hygiene tissue of appropriate dimensions. Suitable dimensions for a flushable wipe or hygiene tissue are: a length between 9 and 25 cm and a width between 7 cm and 15 cm.

An appropriate basis weight for the web is between 30 and 50 g/m$^2$. Two or more webs are combined to a multiply wipe or hygiene tissue, wherein the strength properties of the multiply product is considerably higher than for the individual plies. The webs may be combined to a multiply product either before or after converting it into wipes or hygiene tissue.

The plies are held together by frictional forces and natural hydrogen bonding only, without additional mechanical bonding, such as embossing, needling or hydroentangling, and without adhesive. It has turned out that a dynamic friction coefficient of at least 1.6, preferably at least 1.7 in a wet condition of the plies will be sufficient to accomplish that the plies cling to each other and do not easily delaminate at normal handling of the wet wipe, such as when taking it up from the package, unfolding and using the wet wipe. The friction at average load is measured by the test method given below. By "wet condition" is herein meant the wetted condition of the wipe, in which it has been wetted with 150 weight% of water as calculated on the dry weight of the wipe.

The specific fiber composition of the webs, especially the high amount of pulp fibers, and the hydroentagled structure with fiber ends protruding from the surface of the web, will ensure that the frictional forces between the plies are sufficient for the purpose of this invention.

When the wipe or hygiene tissue is disposed in large quantities of water or is flushed in a sewage system the plies will break apart easily. The individual plies having a relatively low basis weight will be more readily flushable than the laminate.

The wipe or hygiene tissue is impregnated with a wetting composition containing ingredients depending on the intended use of the product. A major proportion of the wetting composition is normally water. Other ingredients may include cleansing agents, skin care agents, bactericides, fungicides, emollients, perfumes, preservatives etc. depending on the intended use.

One use of the wipe or hygiene tissue according to the invention is as a moist toilet paper. As an example a suitable wetting composition in a moist toilet paper may be aqueous based and may contain ingredients like propylene glycol, phenoxy ethanol, coco-glycocide, polyaminopropyl biguanide, dehydroacetic acid, perfume, cocoamidopropyl betaine, chamomilla recutita, bisabolol, citric acid, amylcinnamal, citonellol, hexylcinnamaldehyd, butylphenylmethylpropional and the like.

The wipe or hygiene tissue may contain no or very small amounts of a wet strength agent. A "small amount" is herein defined as up to 0.1 wt% wet strength agent calculated on the dry weight of the wipe or hygiene tissue. High amounts of wet strength agent will deteriorate the flushability of the wipe and make it more difficult to break up and disperse in a sewer.

The moist wipe or hygiene tissue is either individually packed in a sealed package that can be torn open by the user, or a dispenser containing a large number of wipes or tissue that may be dispensed through a dispenser opening in the dispenser.

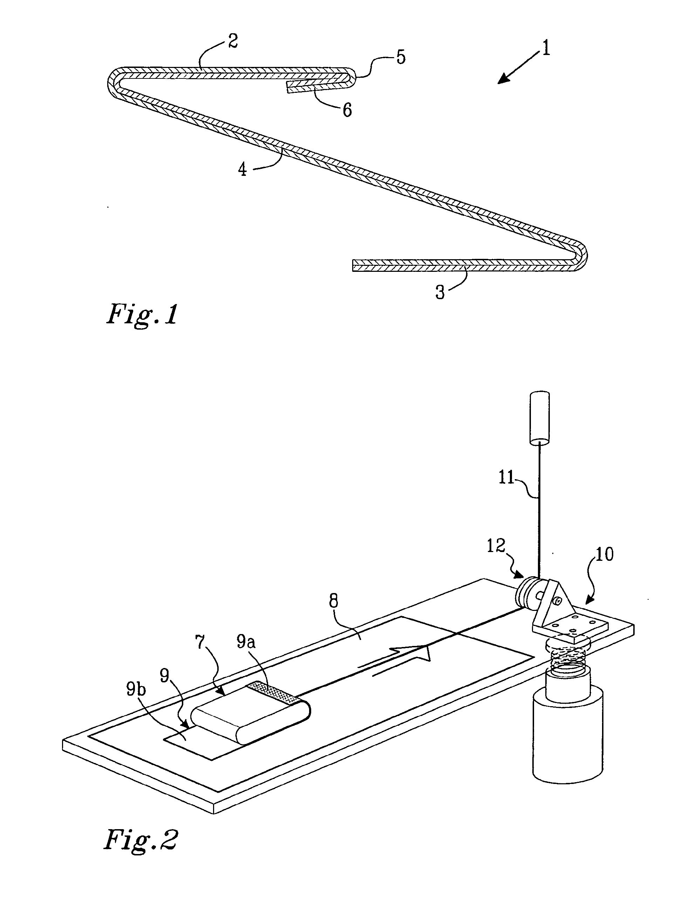

Especially when packaged in a dispenser comprising a plurality of wet wipes or tissue, each individual wipe or hygiene tissue may be folded in a specific manner to facilitate grasping and taking out a single wipe from the dispenser without the risk of delaminating it. Figure 1 illustrates a folded wipe 1 having a leading edge panel 2, a trailing edge panel 3 and an intermediate panel 4 there between. The leading edge panel 2 is provided with an additional fold 5 so as to provide a double-folded leading edge panel lip 6. Such a double-folded leading edge panel lip 6 will facilitate grasping the edge of the wipe without risking delaminating it when taking it out from the dispenser.

The wipes or hygiene tissue may be folded and interleaved in any desired configuration, for example suited for a so called pop-up dispensing system.

The invention will below be exemplified by some embodiments with test results.

EXAMPLES

Test materials were produced as described below.

A fiber dispersion was made from water and a mixture of pulp fibres and manmade staple length fibres. The fibrous web was hydroentangled from one side. The fibrous web was then dewatered by vacuum suction boxes and dried by through-air-drying technique. The fibres used for forming the fibrous web had the following composition:

Ex. 1:  30 wt% polyester from Kuraray Fibers, 1.7 dtex/18 mm;
70 wt% cellulose (bleached sulphate pulp fibres GSM supersoft plus from International Paper). The web was hydroentangled from one side. The web speed was 144 m/min.

Ex. 2:  30 wt% polyester as in Example 1.
70 wt% cellulose as in Example 1.

The web was hydroentangled from one side. The web speed was 144 m/min.

Ex. 3    30 wt% polyester as in Example 1;
70 wt% cellulose as in Example 1.
The web was hydroentangled from one sides. The web speed was 144 m/min.

Evaluations concerning strength properties and friction in wet condition gave the results presented in Table 1 below:

**Table 1**

| Sample | 1 | 2 | 3 |
|---|---|---|---|
| Grammage ($g/m^2$) | 33.7 | 44.6 | 58.7 |
| Thickness ($\mu m$) | 468 | 524 | 628 |
| Wet strength MD (N/m) | 52 | 119 | 235 |
| Wet strength CD (N/m) | 15 | 26 | 58 |
| Force at Average Load Integral (N) | 3.83 | 3.45 | 4.25 |
| Dynamic friction coefficient (wet) | 1.97 | 1.78 | 2.19 |
| Dynamic friction coefficient (dry) | 1.11 | | |

The following test methods were used:

Grammage: SS-EN-ISO 12625-6:2005;
Thickness: SS-EN ISO 12625-3:2005. Deviations from standard method: a) thickness is measured after 25-30 seconds; b) the thickness is measured on five different places on the sample; c) precision dead-weight micrometer sink speed is 1.0 mm/s.
Wet strength: SS-EN ISO12625-5:2005 (measured in water).
Friction: see method described below.

MEASUREMENT OF FRICTION

The method measure the friction coefficient of a material, for example nonwoven or film, under controlled conditions. The friction is indicated as the friction coefficient and measures the dynamic friction. Figure 2 illustrates the apparatus used in the method.
A sample of the test material is attached to a sledge 7. The sample is caused to slide over a friction table 8 having a well defined surface by means of a tensile tester and the force is registered.
The following test equipment was used:

Tensile tester: Instron model 4301
Beam speed : 200 mm/min
Sledge, weight: 198 g (200 $\pm$ 5g)
Balance accuracy: $\pm$ 0.3 g.

*Test procedure*

The samples are prepared by cutting the nonwoven material in pieces 65 x 100 mm and 250 x 150 mm. The samples are then wetted and tested in the wet condition. The longer side shall be in the machine direction. It should be carefully checked that the samples are smooth and not wrinkled or pleated. Fingerprints on the samples should be avoided.
The sledge 7 is weighed and the weight is recorded. The friction table 8 and the sledge 7 are mounted according to the apparatus instruction. A load cell of 10 N is used. Unnecessary touching of the sample and friction table with the fingers should be avoided. A sample 9 (smaller one) is attached to the sledge 7 by taping it at the front edge 9a by double-stick tape. The rear end 9b is free. The larger sample 8 is attached to the rear edge of the friction table by double-stick tape. It should be checked that the sample lies smooth on the friction table. The machine direction of the samples should be in the pulling direction of the apparatus.
The tensile tester is set to zero with the sledge lying on the friction table, without load. The wire 11 of the sledge 7 is led over the friction wheel 12 and the wire is stretched to 0.05 N. The tensile tester is set to zero again. The tensile tester is then started and the sledge 7 is allowed to slide over the friction table 10.

*Calculations*

F= the force at average load (integral) in N registered by the tensile tester and is measured between 45 and 95 mm in the pulling direction
W= weight of sledge (kg)
$\mu_D$= dynamic friction coefficient

$$\mu_D = F/(W \cdot 9.81)$$

**Claims**

1. A flushable moist wipe or hygiene tissue comprising a hydraulically entangled nonwoven material impregnated with a wetting composition, said nonwoven material containing 70-95%, by fibre weight, pulp fibres and 5-30%, by fibre weight, manmade fibres and/or natural fibres with a length of at least 6 mm,
   **characterized in,**
   **that** said moist wipe or hygiene tissue comprises at least two plies of said hydraulically entangled nonwoven material, each ply having a basis weight between 30 and 50 g/m$^2$ and said plies are held together by frictional forces without additional mechanical bonding like embossing, needling and hydroentangling and without adhesive, and that each individual moist wipe or hygiene tissue is folded comprising a leading edge panel (2), a trailing edge panel (3) and optional intermediate panel(-s) (4) there between, wherein the leading edge panel is provided with an additional fold (5) so as to provide a double-folded leading edge panel lip (6).

2. A moist wipe or hygiene tissue as claimed in claim 1,
   **characterized in,**
   **that** said manmade fibres or natural fibres have a fibre length of up to 20 mm.

3. A moist wipe or hygiene tissue as claimed in claim 1 or 2,
   **characterized in,**
   **that** it contains not more than 0.1% by weight, as calculated on the dry weight, of a wet strength agent.

4. A moist wipe or hygiene tissue as claimed in any of the preceding claims,
   **characterized in,**
   **that** the dynamic friction coefficient ($\mu_D$) in wet condition between the plies of said hydraulically entangled nonwoven material is at least 1.6, preferably at least 1.7.

5. A moist wipe or hygiene tissue as claimed in any of the preceding claims,
   **characterized in,**
   **that** it contains at least one ply of tissue paper arranged between plies of said hydraulically entangled nonwoven material.

6. A moist wipe or hygiene tissue as claimed in any of the preceding claims,
   **characterized in,**
   **that** It Is a moist toilet paper.

**Patentansprüche**

1. Spülbares feuchtes Wischtuch oder Hygienetuch mit einem wasserstrahlverfestigten Vliesmaterial, das mit einer benetzenden Zusammensetzung imprägniert ist, wobei das Vliesmaterial nach Fasergewicht 70-95 % Zellstofffasern und nach Fasergewicht 5-30 % künstlich hergestellte Fasern und/oder natürliche Fasern mit einer Länge von mindestens 6 mm enthält,

**dadurch gekennzeichnet, dass**

das feuchte Wischtuch oder Hygienetuch mindestens zwei Lagen des wasserstrahlverfestigten Vliesmaterials aufweist, wobei jede Lage ein Flächengewicht zwischen 30 und 50 g/m$^2$ aufweist und die Lagen ohne zusätzliche mechanische Verbindung, wie zum Beispiel Prägen, Nadeln und Wasserstrahlverfestigen, und ohne ein Haftmittel durch Reibkräfte zusammengehalten werden, und das jedes einzelne feuchte Wischtuch oder Hygienetuch gefaltet ist mit einem Vorderkantenpanel (2), einem Hinterkantenpanel (3) und wahlweise dazwischen einem Zwischenpanel bzw. Zwischenpanels (4), wobei das Vorderkantenpanel mit einer zusätzlichen Faltung (5) bereitgestellt ist, um einen doppelt gefalteten Vorderkantenpanelrand (6) bereitzustellen.

2. Feuchtes Wischtuch oder Hygienetuch nach Anspruch 1,
   **dadurch gekennzeichnet,**
   **dass** die künstlich hergestellten Fasern oder natürlichen Fasern eine Faserlänge von bis zu 20 mm aufweisen.

3. Feuchtes Wischtuch oder Hygienetuch nach Anspruch 1 oder 2,
   **dadurch gekennzeichnet,**
   **dass** es, berechnet in Bezug auf das Trockengewicht, nicht mehr als 0,1 Gew.-% eines Nassfestigkeitsmittels enthält.

4. Feuchtes Wischtuch oder Hygienetuch nach einem der vorstehenden Ansprüche,
   **dadurch gekennzeichnet,**
   **dass** der Gleitreibungskoeffizient ($\mu_D$) im nassen Zustand zwischen den Lagen des hydraulisch verfilzten Vliesmaterials mindestens 1,6 und vorzugsweise mindestens 1,7 beträgt.

5. Feuchtes Wischtuch oder Hygienetuch nach einem der vorstehenden Ansprüche,
   **dadurch gekennzeichnet,**
   **dass** es mindestens eine Lage Tissue-Papier zwischen Lagen des hydraulisch verfilzten Vliesmaterials angeordnet enthält.

6. Feuchtes Wischtuch oder Hygienetuch nach einem der vorstehenden Ansprüche,
   **dadurch gekennzeichnet,**
   **dass** es ein feuchtes Toilettenpapier ist.


## Revendications

1. Lingette humide ou matériau absorbant d'hygiène apte à être jeté dans les toilettes comportant un matériau non tissé entremêlé hydrauliquement imprégné d'une composition humidifiante, ledit matériau non tissé renfermant de 70 à 95 % en poids de fibre, de fibres de pâte de cellulose et de 5 à 30 % en poids de fibres, de fibres artificielles et/ou de fibres naturelles dont la longueur est d'au moins 6 mm,
   **caractérisé en ce que**
   ladite lingette humide ou ledit matériau absorbant d'hygiène comporte au moins deux couches dudit matériau non tissé entremêlé hydrauliquement, chaque couche ayant une masse surfacique comprise entre 30 g/m$^2$ et 50 g/m$^2$ et lesdites couches étant maintenues ensemble par des forces de frottement sans liaison mécanique additionnelle telle que le gaufrage, l'aiguilletage et l'entremêlement hydraulique et sans utilisation d'adhésif, et **en ce que** chaque lingette absorbant individuelle ou chaque matériau absorbant d'hygiène individuel est plié de manière à comporter un panneau (2) de bord avant, un panneau (3) de bord arrière et un panneau intermédiaire optionnel, ou des panneaux intermédiaires optionnels (4) situé, ou situés, entre ceux-ci, le panneau de bord avant étant doté d'un pli supplémentaire (5), de manière à procurer une lèvre (8) de bord avant pliée deux fois.

2. Lingette humide ou matériau absorbant d'hygiène selon la revendication 1,
   **caractérisé en ce que**
   lesdites fibres artificielles ou lesdites fibres naturelles ont une longueur de fibres allant jusqu'à 20 mm.

3. Lingette humide ou matériau absorbant d'hygiène selon la revendication 1 ou 2,
   **caractérisé en ce**
   **qu'**il ou elle ne contient pas plus de 0,1 % en poids, calculé sur le poids à sec, d'un agent de résistance à l'état humide.

4. Lingette humide ou matériau absorbant d'hygiène selon l'une quelconque des revendications qui précèdent,

**caractérisé en ce que**

le coefficient de frottement dynamique ($\mu_o$) à l'état humide entre les couches dudit matériau non tissé entremêlé hydrauliquement est d'au moins 1,6, de préférence d'au moins 1,7.

5. Lingette humide ou matériau absorbant d'hygiène selon l'une quelconque des revendications qui précèdent, **caractérisé en ce**
**qu'**il contient au moins une couche de papier absorbant disposée entre des couches dudit matériau non tissé entremêlé hydrauliquement.

6. Lingette humide ou matériau absorbant d'hygiène selon l'une quelconque des revendications qui précèdent, **caractérisé en ce**
**qu'**il s'agit d'un papier toilette humide.

*Fig.1*

*Fig.2*

REFERENCES CITED IN THE DESCRIPTION

**Patent documents cited in the description**

- EP 0903997 A
- US 3881210 A
- US 6187141 B
- US 6782589 B
- CA 841938